# EUROPEAN PATENT SPECIFICATION

(11) **EP 2 502 586 B1**
(45) Date of publication and mention of the grant of the patent: **03.05.2017**
(21) Application number: 12160687.5
(22) Date of filing: 22.03.2012
(51) Int. Cl.: A61B 17/34, A61B 17/00

(54) **Surgical access port expandable adapter collar assembly**
Erweiterbare Adaptermanschettenanordnung für chirurgischen Zugangsanschluss
Ensemble de collier d'adaptateur extensible de port d'accès chirurgical

(30) Priority: 23.03.2011 US 201161466564 P; 08.02.2012 US 201213368798
(43) Date of publication of application: 26.09.2012
(73) Proprietor: Covidien LP, Mansfield, MA 02048 (US)
(72) Inventor: Okoniewski, Greg, North Haven, CT Connecticut 06473 (US)
(74) Representative: Soames, Candida Jane

(56) References cited:
- EP-A1- 0 950 376
- EP-A1- 2 044 889
- EP-A1- 2 253 283
- EP-A1- 2 289 438
- WO-A1-00/54675
- US-A1- 2002 183 594
- US-A1- 2007 060 884
- US-A1- 2009 221 966
- US-A1- 2010 312 061

## Description

### BACKGROUND

### 1. Technical field

The present disclosure relates to an expandable adapter collar assembly for use with a surgical access port. More particularly, the present disclosure relates to a surgical access port expandable collar assembly including an inflatable collar for use with a surgical access port to allow use of the access port through a large diameter surgical incision having a size greater than the intended design parameters of the surgical access port.

### 2. Background Of Related Art

Certain surgical procedures require forming an incision into the body wall of a patient and positioning a surgical access port through the incision. The body cavity under the body wall of the patient is then filled with insufflation fluids or insufflated to create an expanded working space within the body cavity. The surgical access port provides a sealed passageway for the insertion of surgical instrumentation into the body of the patient while preventing the escape of insufflation fluids. A surgical access port typically has a predetermined outer dimension and is chosen to fit snugly within the size of the surgical incision to prevent the escape of the insufflation fluids.

US 2007/060884 discloses a surgical access port with an expandable collar mounted about a tube.

Some surgical procedures require the removal of a body organ or tissue specimen as part of the surgical procedure. In these instances, the initial surgical incision may not be large enough to withdraw the body organ or tissue sample from the body cavity. In this situation, the body cavity is deflated, the surgical access port is removed from the initial incision and a second or larger diameter surgical incision is made to allow for removal of the body organ or tissue specimen. However, upon reinsertion of the surgical access port into the secondary incision, the size of the surgical access port may not be sufficiently large enough to completely seal within the secondary incision thereby risking the escape of insufflation fluids.

Therefore, there exists a need for an adapter device, usable with a surgical access port having a predetermined outer size, to adjust the overall outer size of the surgical access port and adapter device assembly to conform to the size of the surgical incision. There additionally exists the need for an adapter device usable with a surgical access port to assist the surgical access port in anchoring within the surgical incision.

### SUMMARY

There is disclosed an expandable adapter collar assembly for use in various sized surgical incisions. According to the invention there is provided a surgical access port expanable adapter collar assembly as recited in the independent claim. The expandable collar assembly generally includes an expandable adapter collar defining an inflation chamber and a fluid inflation source in fluid communication with the inflation chamber. Passage of fluid into the inflation chamber from the inflation source moves the expandable adapter collar from a smaller diameter or collapsed, deflated state to a larger diameter expanded state to engage and seal against edges of a surgical incision. The expandable adapter collar assembly includes an inflation tube in fluid communication with the inflation chamber and connectable to an outside source of inflation fluids.

In one embodiment, the expandable adapter collar is doughnut shaped. In an alternative embodiment, the expandable adapter collar is bell shaped. In a specific embodiment, the expandable adapter collar is formed of a stretchable material.

In one particular embodiment, the fluid inflation source is an air source. Alternatively, the fluid inflation sources may be saline or carbon dioxide.

There is also disclosed a surgical access port assembly for use in various sized surgical incisions and generally including an access port having at least one opening for receipt of surgical instruments and an expandable adapter collar assembly mountable around the surgical access port. The expandable adapter collar assembly is movable from a collapsed condition to an expanded condition.

The access port has an hourglass shape defining a waist and the expandable adapter collar assembly is positionable around the waist. The expandable adapter collar assembly includes an inflatable collar defining an inflation chamber and a source of inflation fluids.

In one embodiment, the source of inflation fluids is an air source. In alternative embodiments, the source of inflation fluid is a saline source or a carbon dioxide source.

In one particular embodiment, the inflatable adapter collar is doughnut shaped.

In an alternative embodiment, the inflatable adapter collar is bell shaped. In this embodiment, the inflatable adapter collar includes a cylindrical portion and a flange portion extending distally from the cylindrical portion. The flange portion of the inflatable adapter collar extends around a lower lip of the access port when the inflatable adapter collar is in the expanded condition.

There is further disclosed an exemplary method of securing a surgical access port in an incision having a diameter larger than a waist area of the surgical access port. The exempalry method includes providing an inflatable adapter collar around the waist area of the surgical access port and expanding the inflatable adapter collar from a collapsed condition to a larger expanded condition to engage an incision in a body wall having a diameter larger than the diameter of the waist area of the surgical access port. The exemplary method may additionally include the step of expanding the inflatable adapter collar to engage a lower lip of the surgical access port and an inner surface in the body wall.

### DESCRIPTION OF THE DRAWINGS

Various embodiments of the presently disclosed adapter collars for use with an access port are disclosed herein with reference to the drawings, wherein:
FIG. 1 is a perspective view of a first embodiment of an access port assembly including an inflatable adapter collar and access port;
FIG. 2 is a perspective view, with parts seperated and partially shown in section, of the access port assembly of FIG. 1;
FIG. 3 is a perspective view similar to FIG. 1, partially shown in section;
FIG. 4 is a partial side view, in section, of the access port assembly of FIG. 1 illustrating the inflatable adapter collar inflated with air;
FIG. 5 is a veiw similar to FIG. 4 illustrating the inflatable adapter collar inflated with saline;
FIG. 6 is a perspective view, partially shown in section, of the access port inserted through an incision in tissue;
FIG. 7 is a perspective view, partially shown in section, of the inflatable adapter collar being fitted over the access port and the incision enlarged;
FIG. 8 is a perspective view of the access port assembly being inserted into the enlarged incision;
FIG. 9 is a perspective view, partially shown in section, of the access port assembly with the inflatable adapter collar inflated to fill the enlarged incision;
FIG. 10 is a perspective view of an alternate embodiment of an access port assembly with an alternative inflatable adapter collar and an access port;
FIG. 11 is a perspective view, with parts seperated and partially shown in section, of the access port assembly of FIG. 10;
FIG. 12 is a perspective view, partially shown in section, of the alternative inflatable adapter collar being fitted over the access port and the incision enlarged;
FIG. 13 is a perspective view of the alternative embodiment of the access port assembly being inserted into the enlarged incision;
FIG. 14 is a perspective view, partially shown in section, of the access port assembly with the alternative inflatable adapter collar inflated to fill the enlarged incision; and
FIG. 15 is a partial side view, shown in section, taken along line 15-15 of FIG. 14.

### DETAILED DESCRIPTION OF EMBODIMENTS

Embodiments of the presently disclosed access port assembly will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

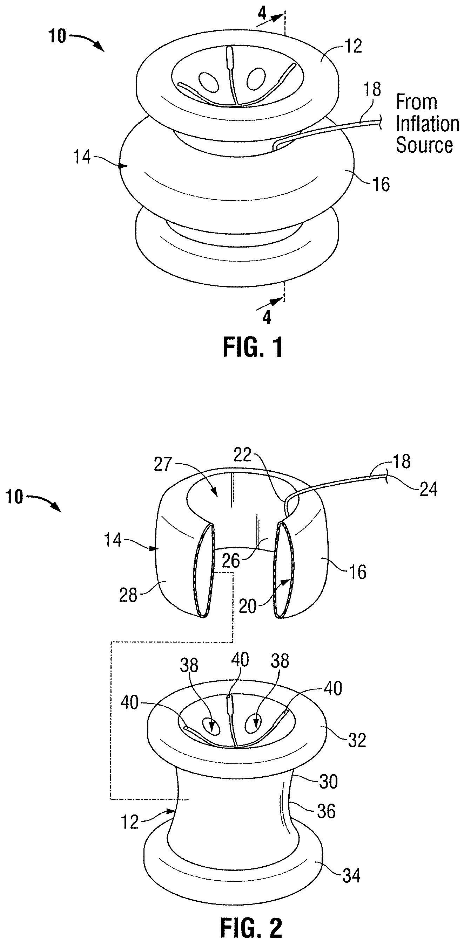

Referring to FIGS. 1-9 and initially to FIGS. 1 and 2, there is disclosed a surgical access port assembly 10 having an access port 12 and an adapter collar assembly 14. Adapter collar assembly 14 is provided to allow use of access port 12 in an incision having an opening greater than the design parameters of the access port by filling the space between the access port and an inner edge of the incision. Adapter collar assembly 14 includes a generally doughnut shaped expandable collar 16 and an inflation tube 18.

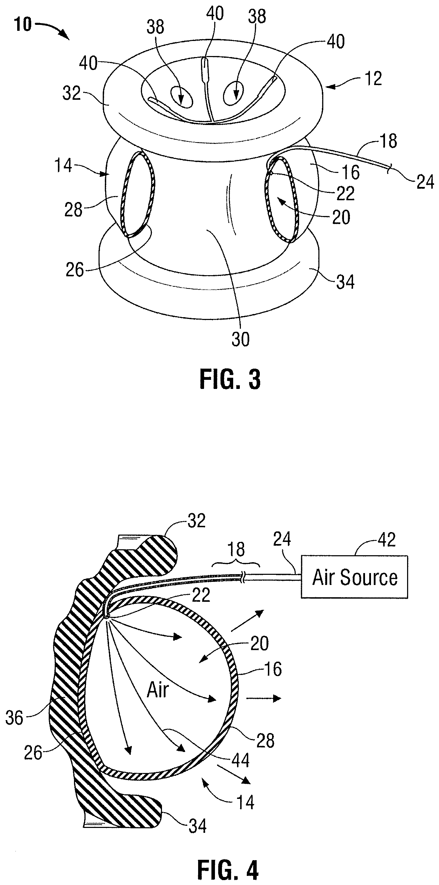

As best shown in FIGS. 2 and 3, expandable collar 16 is hollow and defines an inflation chamber 20. A distal end 22 of inflation tube is in fluid communication with inflation chamber 20 and a proximal end 24 of inflation tube 18 is connectable to a source of inflation fluid in a manner described in more detail hereinbelow. Expandable collar 16 may be formed from a variety of materials including stretchable materials such as, for example, polymers, rubber, etc. The stretchable material allows expandable collar 16 to lie flat against access port 12 in a deflated state prior to inflation and can be inflated and expanded away from access port 12 to adjust the size of expandable collar 16 to the incision size in order to secure access port 12 within the incision. Alternatively, expandable collar 16 may be formed from non-stretchable materials such as, for example, plastics, etc. In this situation, expandable collar 16 may include folds, etc. to allow expandable collar 16 to lie flat against access port 12 during insertion into the incision.

As best shown in FIG. 2, expandable collar 16 includes an inwardly projecting or inner surface 26 and an outwardly projecting or outer surface 28. Inner surface 26 defines a central opening 27 for receipt of access port 12. Access port 12 includes an hourglass shaped center portion 30, a proximal or upper lip 32 and a distal or lower lip 34. Hourglass shaped center portion 30 is narrowest at a waist 36. With reference to FIGS. 2 and 3, access port 12 includes a plurality of ports 38 which extends through access port 12 for the receipt and passage of surgical instrumentation. Access port 12 additionally includes a plurality of longitudinally extending slits 40 which also extend through access port 12 for receipt of surgical instrumentation.

Expandable collar 16 is assembled to access port 12 by positioning expandable collar 16 adjacent access port 12 (FIG. 2) and stretching expandable collar 16 such that it fits over upper lip 32 of access port 12. Thereafter, expandable collar 16 is moved along access port 12 to a position adjacent and surrounding hourglass shaped center portion 30 (FIG. 3). In this manner, the expandable collar 16 may be selectively attached and detached to the access port 12.

As best shown in FIG. 4, in this position, inner surface 26 of expandable collar 16 engages and lies flush against waist 36 of hourglass shaped center portion 30 of access port 12. As noted herein above, access port assembly 10 includes, or is connectable to, a fluid source such as, for example, fluid air source 42. Fluid air source 42 is provided in order to inject air 44 through inflation tube 18 and into inflation chamber 20 in order to move expandable collar 16 from the deflated to an inflated condition. Specifically, air source 42 is connected to proximal end 24 of inflation tube 18.

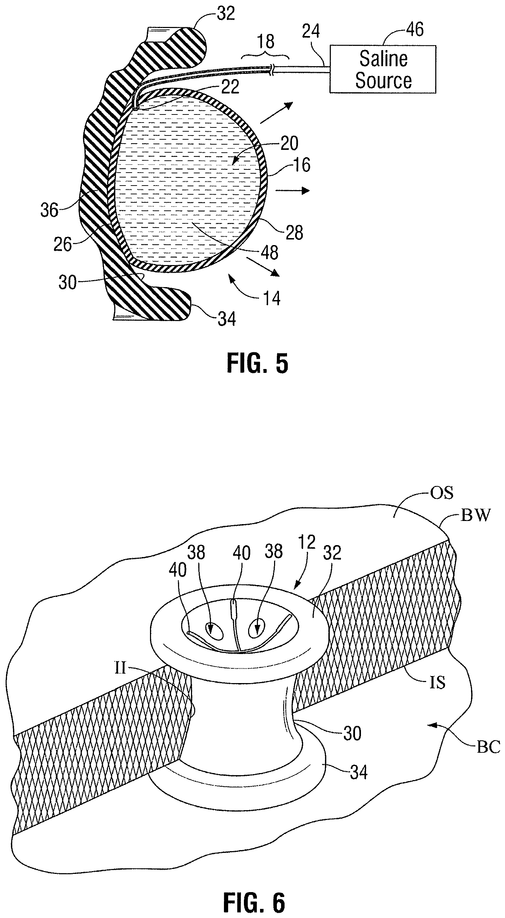

Referring for the moment to FIG. 5, alternatively, the fluid source may be a saline source 46 which is connectable to proximal end 24 of inflation tube 18. Saline source 46 is provided to supply a source of saline 48 into inflation chamber 20 of expandable collar 16 to move expandable collar 16 from the deflated to an inflated condition. It should be noted that carbon dioxide and other standard operating room fluid sources may also be used as inflation sources.

Referring now to FIGS. 6-9, and initially to FIG. 6, the use of access port assembly 10 will now be described. A surgical procedure is performed by making a first or initial incision II through a body wall BW to access a body cavity BC. Access port 12 is inserted into initial incision II such that hourglass center portion 30 engages the inner surfaces of the initial incision II. Upper lip 32 engages outer surface OS body wall BW and lower lip 34 engages inner surface IS body wall BW to secure access port 12 within body wall BW. Thereafter, a surgical procedure may be performed by inserting surgical instrumentation through ports 38 or slits 40.

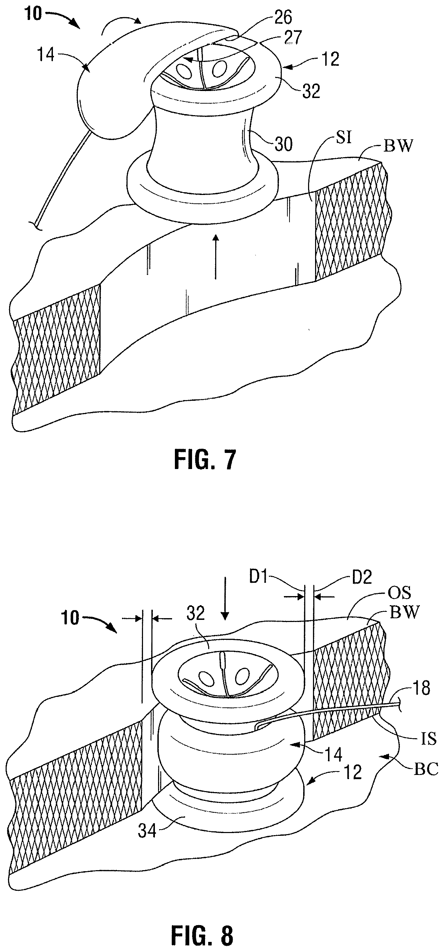

Referring now to FIGS. 7 and 8, once the first surgical procedure has been performed, access port 12 may be removed from body wall BW and initial incision II enlarged to form a larger secondary insertion SI (FIG. 7). As best shown in FIG. 7, adapter collar assembly 14 is then positioned over access port 12 and seated against waist portion 30 of access port 12. Specifically, access port 12 is inserted through central opening 27 in expandable collar 16.

With specific reference to FIG. 8, access port assembly 10 is then inserted into secondary incision SI. As shown, diameter D2 of secondary incision SI is greater than the diameter D1 of initial incision II or upper and lower lips 32 and 34, respectively, of access port 12. Adapter collar assembly 14 is in a deflated state and lies against access port 12. Inflation tube 18 extends outward of body wall BW.

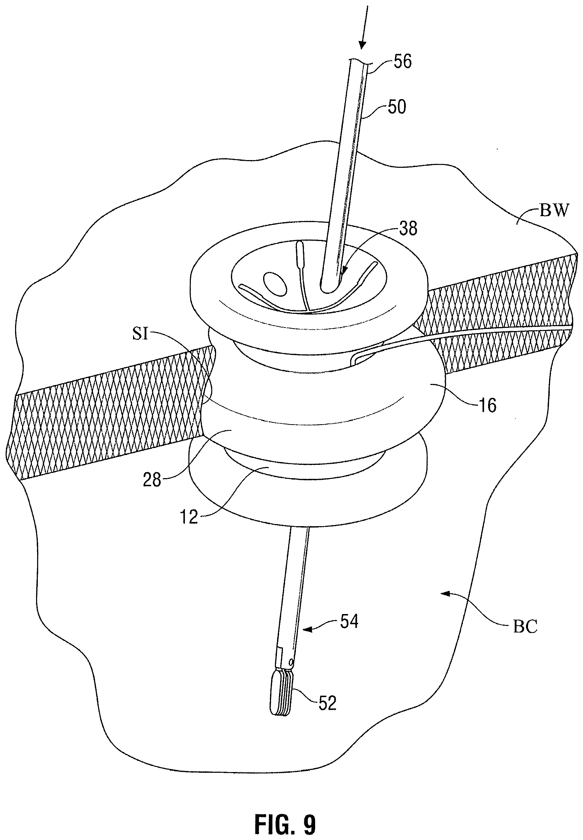

Referring now to FIG. 9, fluid source 42 (FIG. 4) is then actuated to inject fluid into inflation chamber 20 of expandable collar 16 to move expandable collar 16 from the contracted or deflated state to an expanded or inflated state wherein outer surface 28 of expandable collar 16 engages the inner surfaces of the secondary incision SI to secure access port assembly 10 within body wall BW. At this point, a surgical instrument such as, for example, surgical instrument 50 is inserted through port 38 to position a surgical tool 52 at a distal end 54 of surgical instrument 50 within body cavity BC. Thereafter, actuation structure (not shown) located at proximal end 56 of surgical instrument 50 may be actuated to operate surgical tool 52. Once the secondary surgical procedure has been completed, fluid air source 42 (FIG. 4) may be again actuated to deflate expandable collar 16 allowing removal of access port assembly 10 from secondary incision SI. In this manner, access port assembly 10 and, in particular expandable collar 16, allows use of access port 12 in varying diameter surgical incisions.

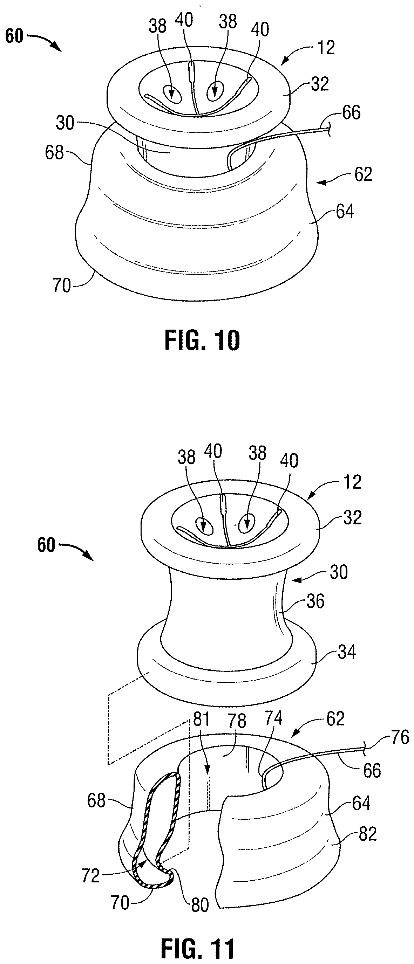

Referring now to FIGS. 10-15, and initially with regard to FIGS. 10 and 11, there is disclosed an alternative embodiment of an access port assembly 60 including access port 12 and an adapter collar assembly 62. Access port 12 is identical to access port 12 described hereinabove including hourglass shaped center portion 30, upper lip 32 and lower lip 34. As noted hereinabove, hourglass shaped center portion 30 includes a narrow waist 36 and through ports 38 for receipt of surgical instrumentation along with longitudinal slits 40.

Adapter collar assembly 62 generally includes an expandable bell shaped collar 64 and an inflation tube 66. As best shown in FIG. 11, bell shaped collar 64 includes a proximal or upper cylindrical portion 68 and a distal or lower flange portion 70 which together define an inflation chamber 72. A distal end 74 of inflation tube 66 is in fluid communication with inflation chamber 72 and a proximal end 76 of inflation tube 66 is connectable to a source of fluid in a manner described in more detail hereinbelow.

It should be noted that bell shaped collar 64 is formed of a material similar to that described herein above with regard to doughnut shaped collar 16. Lower flange portion 70 is sufficiently flexible that, upon inflation, it can wrap around and under lower lip 34 of access port 12. Specifically, an inner surface 78 of cylindrical portion 68 is configured to engage hourglass center portion 30 of access port 12 while an inner surface 80 of lower flange portion 70 is configured to engage and surround lower lip 34 of access port 12. Inner surfaces 78 and 80 of cylindrical portion 68 and lower lip 34, respectively, define a central opening 81 for receipt of access port 12. An outer surface 82 of expandable collar 64 is engaged within incision formed in the body wall of a patient in a manner described in more detail hereinbelow.

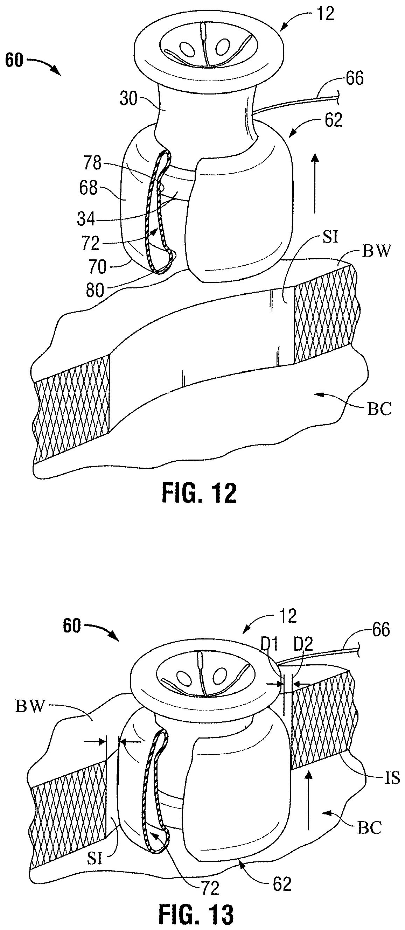

Referring now to FIGS. 12-15, the use of access port assembly 60 will now be described. As with the procedure described herein above, surgical access port 12 is utilized to perform a surgical procedure through an initial incision II (FIG. 6). Thereafter, in order to remove a specimen from body cavity BC, access port 12 is removed and expandable collar 64 is fitted over access port 12. With specific reference to FIG. 12, adapter collar assembly 62 is slid over access port 12 such that inner surface 78 engages hourglass shaped portion 30 of access port 12 and inner surface 80 of distal flange 70 engages lower lip 34 of access port 12.

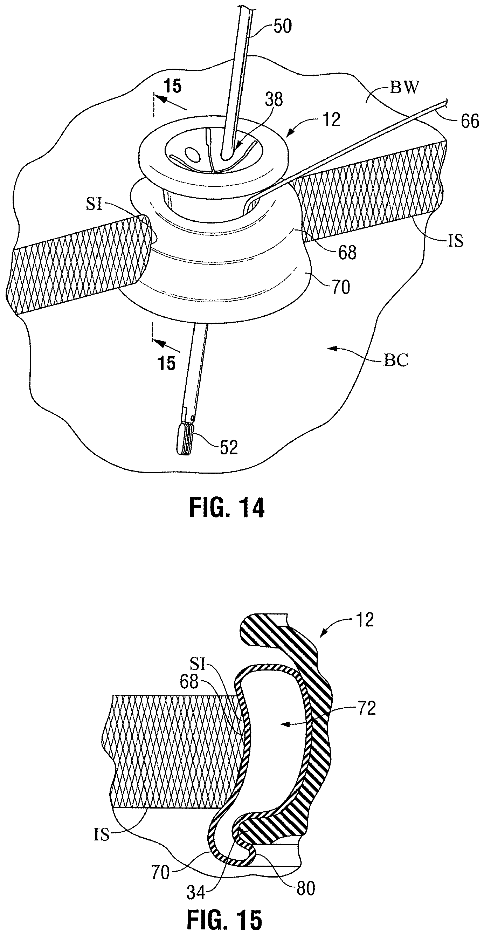

Referring now to FIG. 13, as noted herein above, a secondary incision SI is formed having a diameter D2, which is greater then the diameter D1 of intitial incision II. Thereafter, access port assembly 60 is inserted into secondary incision SI with bell shaped expandable collar 64 in the deflated state. A source of fluids such as, for example, source of fluid 42 (FIG. 4) is connected to inflation tube 66 and used to force an inflation fluid into inflation chamber 72 to expand bell shaped expandable collar 64 from the deflated state to an expanded state engaging secondary incision SI in body wall BW (FIG. 14).

As best shown in FIGS. 14 and 15, lower flange portion 70 of bell shaped expandable collar 64 engages inner surface IS of body wall BW to prevent access port assembly 60 from backing out of body wall BW during a surgical procedure. With specific reference to FIG. 15, inner surface 80 of lower flange portion 70 engages and wraps around lower lip 34 of access port 12 to further secure bell shaped expandable collar 64 about access port 12 during a surgical procedure.

Referring back for the moment to FIG. 14, similar to the surgical procedure described hereinabove with regard to access port assembly 10, surgical instrument 50 may be inserted through port 38 in access port 12 to position tool 52 within body cavity BC to thereby remove a specimen (not shown) from within body cavity BC. Once a specimen has been grasped by tool 52 of surgical instrument 50, fluid within inflation chamber 72 may be removed through inflation tube 66 to deflate bell shaped expandable collar 64 and allow removal of access port assembly 60 along with surgical instrument 50 and any grasped specimen through secondary incision SI.

In this manner, access port assembly 60 provides a variable diameter access port for use with various sized incisions and includes structure to facilitate additional anchoring of access port assembly 60 within an enlarged surgical incision and allows use of a standard sized surgical access port through a larger than indicated incision.

It will be understood that various modifications may be made to the embodiments disclosed herein. For example, the disclosed expandable collar may have other shapes including a full hourglass shape, a rectangular cross section, etc. Further, inflation fluids may be provided into the inflation chamber via a syringe. Additionally, the disclosed expandable collars may be formed from compressible materials rather than an inflatable chamber and be formed of foam, solid rubber, etc. Therefore, the above description should not be construed as limiting, but merely as exemplifications of particular embodiments. Those skilled in the art will envision other modifications within the scope of the claims appended hereto.

## Claims

1. A surgical access port expandable adapter collar assembly for use in various sized surgical incisions comprising:
an access port (12) having at least one opening (38) for receipt of surgical instruments; and
an expandable collar assembly (14, 62) mountable around the surgical access port, wherein the access port (12) has design parameters to fit a first size of incision without the expandable collar assembly (14, 62) and the expandable collar assembly (14, 62) being movable when mounted around the access port (12) from a collapsed condition to an expanded condition to fit the assembly to a second size of incision having an opening greater than the design parameters of the access port (12).

2. The surgical access port expandable adapter collar assembly as recited in claim 1, wherein the access port (12) has an hourglass shape defining a waist (36) and the expandable collar assembly (14, 62) is positionable around the waist (36).

3. The surgical access port expandable adapter collar assembly as recited in claim 1 or claim 2, wherein the expandable collar assembly (14, 62) includes an inflatable collar (16, 64) defining an inflation chamber (20) and a source (42, 46) of inflation fluids.

4. The surgical access port expandable adapter collar assembly as recited in claim 3, wherein the source of inflation fluids is an air source (42); and/or wherein the source of inflation fluids is a saline source (46); and/or wherein the source of inflation fluids is a carbon dioxide source.

5. The surgical access port expandable adapter collar assembly as recited in claim 1, wherein the inflatable collar (16) is doughnut shaped; or wherein the inflatable collar (64) is bell shaped.

6. The surgical access port expandable adapter collar assembly as recited in claim 5, wherein the inflatable collar (64) includes a cylindrical portion (68) and a flange portion (70) extending distally from the cylindrical portion (68).

7. The surgical access port expandable adapter collar assembly as recited in claim 6, wherein the flange portion (70) of the inflatable collar (64) extends around a lower lip (34) of the access (12) when the inflatable collar (64) is in the expanded condition.

8. The expandable adapter collar assembly as recited in claim 3, wherein the inflation source (42, 46) includes an inflation tube (18, 66) in fluid communication with the inflation chamber (20).

9. The expandable adapter collar assembly as recited in any preceding claim, wherein the expandable collar (16, 64) is formed of a stretchable material.

## Patentansprüche

1. Expandierbare Adapterkragenanordnung für einen chirurgischen Zugangsport zur Verwendung bei verschieden bemessenen chirurgischen Einschnitten, umfassend:
einen Zugangsport (12), der mindestens eine Öffnung (38) zur Aufnahme von chirurgischen Instrumenten aufweist; und
eine expandierbare Kragenanordnung (14, 62), die um den chirurgischen Zugangsport herum befestigbar ist,
wobei der Zugangsport (12) Designparameter aufweist, damit er zu einer ersten Einschnittgröße ohne die expandierbare Kragenanordnung (14, 62) passt, und wobei die expandierbare Kragenanordnung (14, 62) von einem kollabierten Zustand in einen gedehnten Zustand beweglich ist, wenn sie um den Zugangsport (12) herum befestigt wird, um die Anordnung auf eine zweite Einschnittgröße anzupassen, deren Öffnung größer als die Designparameter des Zugangsports (12) ist.

2. Expandierbare Adapterkragenanordnung für einen chirurgischen Zugangsport nach Anspruch 1, wobei der Zugangsport (12) eine Sanduhrform aufweist, die eine Taille (36) definiert, und wobei die expandierbare Kragenanordnung (14, 62) um die Taille (36) herum positionierbar ist.

3. Expandierbare Adapterkragenanordnung für einen chirurgischen Zugangsport nach Anspruch 1 oder Anspruch 2, wobei die expandierbare Kragenanordnung (14, 62) einen aufblasbaren Kragen (16, 64), beinhaltet, der eine Inflationskammer (20) und eine Quelle (42, 46) von Inflationsflüssigkeiten definiert.

4. Expandierbare Adapterkragenanordnung für einen chirurgischen Zugangsport nach Anspruch 3, wobei die Quelle von Inflationsflüssigkeiten eine Luftquelle (42) ist; und/oder wobei die Quelle von Inflationsflüssigkeiten eine Kochsalzquelle (46) ist; und/oder wobei die Quelle von Inflationsflüssigkeiten eine Kohlendioxidquelle ist.

5. Expandierbare Adapterkragenanordnung für einen chirurgischen Zugangsport nach Anspruch 1, wobei der aufblasbare Kragen (16) eine Kreisringform aufweist; oder wobei der aufblasbare Kragen (64) glockenförmig ist.

6. Expandierbare Adapterkragenanordnung für einen chirurgischen Zugangsport nach Anspruch 5, wobei der aufblasbare Kragen (64) einen zylindrischen Abschnitt (68) und einen Flanschabschnitt (70) aufweist, der sich distal vom zylindrischen Abschnitt (68) erstreckt.

7. Expandierbare Adapterkragenanordnung für einen chirurgischen Zugangsport nach Anspruch 6, wobei sich der Flanschabschnitt (70) des aufblasbaren Kragens (64) um eine untere Lippe (34) des Zugangs (12) herum erstreckt, wenn sich der aufblasbare Kragen (64) im gedehnten Zustand befindet.

8. Expandierbare Adapterkragenanordnung nach Anspruch 3, wobei die Inflationsquelle (42, 46) ein Inflationsrohr (18, 66) in Fluidkommunikation mit der Inflationskammer (20) beinhaltet.

9. Expandierbare Adapterkragenanordnung nach einem der vorstehenden Ansprüche, wobei der expandierbare Kragen (16, 64) aus einem dehnbaren Material gebildet ist.

## Revendications

1. Ensemble de collier d'adaptateur extensible d'orifice d'accès chirurgical pour utilisation dans des incisions chirurgicales de diverses tailles, comprenant :
un orifice d'accès (12) ayant au moins une ouverture (38) pour recevoir des instruments chirurgicaux ; et
un ensemble de collier extensible (14, 62) montable autour de l'orifice d'accès chirurgical, dans lequel l'orifice d'accès (12) a des paramètres de construction pour s'adapter à une première taille d'incision sans l'ensemble de collier extensible (14, 62) et l'ensemble de collier extensible (14, 62) pouvant se déplacer lorsqu'il est monté autour de l'orifice d'accès (12) d'un état replié à un état en extension pour adapter l'ensemble à une seconde taille d'incision ayant une ouverture supérieure à celle des paramètres de construction de l'orifice d'accès (12).

2. Ensemble de collier d'adaptateur extensible d'orifice d'accès chirurgical selon la revendication 1, dans lequel l'orifice d'accès (12) a une forme de sablier définissant une partie centrale (36) et l'ensemble de collier extensible (14,62) peut être positionné autour de la partie centrale (36).

3. Ensemble de collier d'adaptateur extensible d'orifice d'accès chirurgical selon la revendication 1 ou la revendication 2, dans lequel l'ensemble de collier extensible (14, 62) comprend un collier gonflable (16, 64) définissant une chambre de gonflage (20) et une source (42, 46) de fluides de gonflage.

4. Ensemble de collier d'adaptateur extensible d'orifice d'accès chirurgical selon la revendication 3, dans lequel la source de fluides de gonflage est une source d'air (42); et/ou dans lequel la source de fluides de gonflage est une source saline (46) ; et/ou dans lequel la source de fluides de gonflage est une source de dioxyde de carbone.

5. Ensemble de collier d'adaptateur extensible d'orifice d'accès chirurgical selon la revendication 1, dans lequel le collier gonflable (16) est en forme de beignet ; ou dans lequel le collier gonflable (64) est en forme de cloche.

6. Ensemble de collier d'adaptateur extensible d'orifice d'accès chirurgical selon la revendication 5, dans lequel le collier gonflable (64) comprend une partie cylindrique (68) et une partie bridée (70) s'étendant de manière distale de la partie cylindrique (68).

7. Ensemble de collier d'adaptateur extensible d'orifice d'accès chirurgical selon la revendication 6, dans lequel la partie bridée (70) du collier gonflable (64) s'étend autour d'une lèvre inférieure (34) de l'accès (12) lorsque le collier gonflable (64) est à l'état dilaté.

8. Ensemble de collier d'adaptateur extensible selon la revendication 3, dans lequel la source de gonflage (42, 46) comprend un tube de gonflage (18, 66) en communication fluidique avec la chambre de gonflage (20).

9. Ensemble de collier d'adaptateur extensible selon l'une quelconque des revendications précédentes, dans lequel le collier extensible (16, 64) est formé d'un matériau étirable.
